# EUROPEAN PATENT APPLICATION

(11) **EP 4 134 028 A1**
(43) Date of publication of application: **15.02.2023**
(21) Application number: 22182599.5
(22) Date of filing: 01.07.2022
(51) Int. Cl.: A61B 18/12, A61B 18/14, F16J 15/02, F16J 15/10, H05K 5/00, A61B 18/00, A61B 17/00, A61B 90/00

(54) **SURGICAL DEVICE SEAL**

(30) Priority: 10.08.2021 US 202163231281 P
(71) Applicant: Gyrus Medical Limited, Cardiff CF3 0LT (GB)
(72) Inventor: BLAKE, Nathan Philip, Cardiff, CF3 0LT (GB)
(74) Representative: Wallin, Nicholas James

(57) **Abstract**

A surgical instrument is disclosed, which is configured to be releasably connectable to a hand-piece. The surgical instrument includes electrical contacts configured to be electrically connected to the hand-piece. The surgical instrument further includes a sealing member having sealing elements. At least one sealing element of the plurality of sealing elements is formed of an elastomeric material and is configured to form a seal around one the electrical contacts when the surgical instrument is connected to the hand-piece, such that the electrical contact is electrically isolated from other electrical contacts on the surgical instrument. At least one sealing element is configured such that a force required to connect the surgical instrument to the hand-piece is not reliant on a bulk compression of the elastomeric material.

## Description

### Technical Field

Embodiments of the present invention described herein relate to a surgical device, and in particular to an electrosurgical device wherein a disposable instrument is connectable to a hand-piece via a connection hub, the connection hub facilitating an electrical and mechanical connection therebetween.

### Background of the Invention

Electrosurgical instruments provide advantages over traditional surgical instruments in that they can be used for coagulation and tissue sealing purposes. One such prior art arrangement is known from US 5,904,681 which describes a surgical instrument including a mechanical cutting portion, such as a rotary blade or burr, and a radio frequency (RF) cutting and/or cauterizing portion comprising an electronic surgical device which operates in bipolar mode.

Another prior art arrangement is known from US 9,017,851 which describes an apparatus for powering a medical device, including a protective layer covering a battery pack and a connection feature. The connection feature provides a fluid tight seal, for example by having an electrode which may pierce the protective layer to establish electrical communication with the medical device from within the protective layer, thereby allowing a non-sterile battery pack to deliver power to a sterile medical device.

Another prior art arrangement is known from EP0746251B1 which describes an integrated catheter assembly including a catheter having an irrigation fluid lumen, a distal tip portion having electrodes, and a needle extending through the catheter lumen and a lumen in the distal tip portion. The catheter is carried by a needle hub structure which provides a sealing entrance for a needle that can be displaced between extended and retracted positions while being electrically isolated.

Another prior art arrangement is known from US2009/221955A1 which describes an ablative apparatus having a distal end at which an ablation probe driven by a transducer may be vibrated to ablate tissues.

### Summary of the Invention

Embodiments of the present invention provide an improved surgical system having an instrument and a handpiece with a sealing component therebetween. The instrument has an elongate shaft comprising a connection hub and a known end effector capable of different operations including mechanical cutting or shaving of tissue, and/or electrosurgical ablation, sealing and/or coagulation of tissue. The surgical instrument can be connected to a hand-piece for provision of fluid, mechanical power and/or radiofrequency (RF) electrical signals. A single hand-piece can be re-used for multiple surgeries while the surgical instrument may be disposable, meaning that it might only be used for a single surgical operation performed on a single patient, before being disposed.

The focus of this disclosure is on the connection between the hand-piece and the surgical instrument, which must be capable of transferring mechanical as well as electrical power. The instrument disclosed herein facilitates the inclusion of electrical connections in the connection hub so that it may receive electrical signals and power from the hand-piece, while ensuring safe operation of the device. In particular, the instrument and the hand-piece comprise a plurality of mutually cooperating electrical contacts for the communication of electrical power. Due to the saline rich environment in which the surgical device is operated, electrical contacts on the same component of the system (e.g. two contacts on the instrument) need to be isolated from one another to prevent electrical shorting. To achieve this, electrical contacts on the surgical instrument can be provided with a seal that acts around its perimeter, thereby isolating its electrical connection with a corresponding electrical contact on the hand-piece from other electrical contacts on the instrument and the hand-piece.

In view of the above, from one aspect the invention provides a surgical instrument configured to be releasably connectable to a hand-piece, the surgical instrument comprising:
an end effector for performing surgical operations;
a plurality of electrical contacts configured to be electrically connected to the hand-piece,
a sealing member comprising a plurality of sealing elements, wherein at least one sealing element of the plurality of sealing elements comprises an elastomeric material and is configured to form a seal around one of the plurality of electrical contacts when the surgical instrument is connected to the hand-piece, such that the electrical contact is electrically isolated from other electrical contacts on the surgical instrument,
wherein the at least one sealing element is configured such that the elastomeric material deflects into a position to form the seal and does not undertake a bulk compression of the elastomeric material to form the seal.

A 'bulk compression' of the elastomeric material refers to the exertion of compressive stresses throughout the elastomeric material. This type of deformation requires a relatively high force in order to compress the elastomeric material between the surgical instrument and the hand-piece. In contrast, the arrangement above does not rely on such a compression of the elastic material to form the seal. Therefore, such an arrangement provides an advantageous surgical instrument which requires an appropriate and lesser degree of force to connect it to a hand-piece.

The sealing element may comprise a tapered cavity configured to facilitate deflection of the sealing element when the surgical instrument is connected to the hand-piece. By providing a tapered cavity, connection of the surgical instrument and the hand-piece may be facilitated by the displacement of air in the cavity, instead of by bulk compression of elastomeric material. This significantly reduces the force required to deform the sealing element between the surgical instrument and the hand-piece.

The sealing element may comprise a deflectable portion. The deflectable portion may be configured to deflect when compressed between the surgical instrument and the hand-piece. The deflectable portion may be configured to bend in response to a compression force exerted on the sealing member when the surgical instrument is connected to the hand-piece. It will be appreciated that bending a wall of elastomeric material may require less force than compressing the elastomeric material.

The sealing element may comprise an opening through which one of the plurality of electrical contacts is configured to electrically connect to the hand-piece. The opening may be comprised in the narrowest end of the deflectable portion. This arrangement can provide a space for accommodating the electrical contacts while minimising the size of the opening through which the electrical connection is made. This helps to provide a more effective seal to isolate a first electrical connection between the instrument and the hand-piece from a second electrical connection between the instrument and the hand-piece, to thereby prevent electrical shorting when the instrument and hand-piece are being operated in a region of electrically conductive fluid.

The deflectable portion may comprise a sloped wall. The sloped wall may have a thickness that is substantially uniform along at least 50%, preferably at least 70%, of the height of the deflectable portion from the opening. An internal diameter of the deflectable portion may decrease gradually towards the opening. This provides an advantageous sealing element wherein the force required to deflect the deflectable portion is reduced.

The deflectable portion may comprise a frustoconical portion. The sealing member may comprise a substantially cylindrical body portion. The frustoconical portion may at least partially protrude from a flat end face of the cylindrical body portion. This provides a sealing element which can deflect uniformly around its circumference in response to being compressed between the surgical instrument and the hand-piece, which provides a more effective and durable seal.

The sealing element may comprise a fixed portion. The fixed portion may be configured to be mounted within the sealing member. The fixed portion is preferably substantially cylindrical. This provides a way to mount the sealing element in the sealing member which simplifies manufacture and assembly.

The surgical device may comprise at least five pairs of corresponding electrical contacts. The sealing member may comprise five sealing elements. The sealing elements may be configured to isolate the pairs of corresponding electrical contacts from one another when the surgical instrument is connected to the hand-piece. It will be appreciated that there may be more or fewer sealing elements or corresponding pairs of electrical contacts, and that such devices could still benefit from the advantageous arrangement described above.

The sealing member may comprise a circular array of sealing elements. At least one bridge portion may link neighbouring sealing elements in the array. A single elastomeric moulding may comprise the sealing elements and the at least one bridge portion. This allows the array of sealing elements to be provided in a single component, thereby simplifying and speeding up manufacture and assembly.

Another aspect of the invention provides a surgical device comprising a hand-piece, a surgical instrument and a sealing member. The hand-piece comprises a first set of electrical contacts. The surgical instrument is configured to be releasably connectable to the hand-piece along a connection axis and comprises a second set of electrical contacts. Each of the second set of electrical contacts corresponds to one of the first set of electrical contacts to form pairs of corresponding electrical contacts configured to provide electrical connections between the surgical instrument and the hand-piece. The sealing member is configured to be disposed between the hand-piece and the surgical instrument. The sealing member comprises a plurality of sealing elements. The sealing elements comprise an elastomeric material. At least one of the plurality of sealing elements is configured to form a seal around a pair of corresponding electrical contacts.

At least one sealing element may be configured such that when the surgical instrument is connected to the hand-piece, the sealing element is disposed around at least one pair of corresponding electrical contacts to thereby seal the pair from at least one other pair of corresponding electrical contacts. The at least one sealing element may be configured such that a force required to connect the surgical instrument to the hand-piece is not reliant on a bulk compression of the elastomeric material.

### Brief Description of the Drawings

Further features and advantages of the present invention will become apparent from the following description of embodiments thereof, presented by way of example only, and by reference to the drawings, in which:
Figure 1 is a schematic diagram of a surgical system including a surgical instrument according to an embodiment;
Figure 2 is a perspective view of a surgical device according to an embodiment;
Figure 3 is a perspective view of a sealing member;
Figure 4 is a cross sectional view of the sealing member in Figure 3;
Figure 5 is a perspective view of a sealing member according to an embodiment;
Figure 6 is a cross sectional view of the sealing member in Figure 5.

### Detailed Description

A surgical instrument is described herein in the context of a surgical system for performing procedures such as ablation, sealing, resection and coagulation of tissues. The apparatus includes an electrosurgical generator capable of providing a mechanical and/or RF output. Such outputs may be controlled by various user inputs such as push buttons and switches, with the levels of various settings and other information being indicated on a display. The generator is connected to an electrosurgical device via a connection cord capable of transmitting power and RF signals thereto. Additionally, an irrigation and suction source is provided, capable of drawing fluid from, or providing fluid to, the electrosurgical device through tubes. The connection cord and tubes are connected to a hand-piece of the electrosurgical device. The hand-piece provides a means with which a practitioner may manipulate the electrosurgical device, and it may have further user input devices thereon for controlling the mechanical and/or RF output of the generator.

The surgical instrument, which is described herein by way of example as an electrosurgical instrument, can be releasably connected to the hand-piece. The hand-piece includes electrical contacts which correspond to electrical contacts provided on the surgical instrument to facilitate the transmission of electrical power therebetween. Between the surgical instrument and the hand-piece is a sealing member having a number of sealing elements which may correspond to the number of pairs of corresponding electrical contacts. The sealing elements may be formed of an electrically insulative material, such as an elastomeric material. Each sealing element may comprise an opening through which a pair of electrical contacts may be electrically connected. The shape of the sealing elements is configured such that when the instrument is connected to the hand-piece, the sealing elements form a seal around the pairs of corresponding electrical contacts to isolate the pairs from one another. The shape of the sealing elements is configured such that the force required to connect the instrument to the hand-piece is reliant on a force required to deflect a deflectable portion of the sealing element rather than on the force required to provide a bulk compression of the elastomeric material.

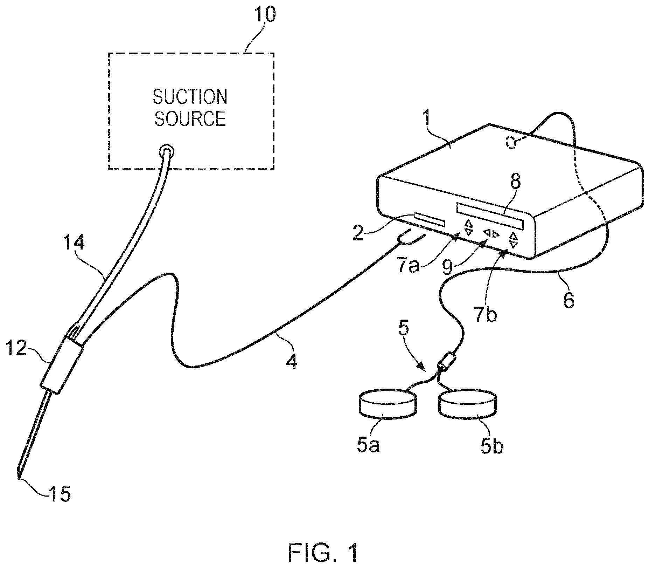

Referring to the drawings, Figure 1 shows a surgical system including an electrosurgical generator 1 having an output socket 2 providing a radio frequency (RF) output, via a connection cord 4, for an electrosurgical device 12. The device 12 has an end effector 15, and irrigation and suction tubes 14 which are connected to an irrigation fluid and suction source 10. Activation of the generator 1 may be performed from the device 12 via a hand-switch (not shown) on the device 12, or by means of a footswitch unit 5 connected separately to the rear of the generator 1 by a footswitch connection cord 6. In the illustrated embodiment, the footswitch unit 5 has two footswitches 5a and 5b for selecting a coagulation mode or a cutting or vaporisation (ablation) mode of the generator 1 respectively, although in some embodiments of the electrosurgical device 12 described herein it is envisaged that only one or other of the coagulation or ablation modes would be used, with cutting being provided mechanically by way of a rotating tube having a sharpened cut-out portion. The generator front panel has push buttons 7a, 7b for respectively setting ablation (cutting) or coagulation power levels, which are indicated in a display 8. Push buttons 9 are provided as an alternative means for selection between the modes of operation. In an alternative arrangement, the device 12 may include means to generate electrosurgical signals, for example using an electrosurgical generator that can be an integral part of the device 12.

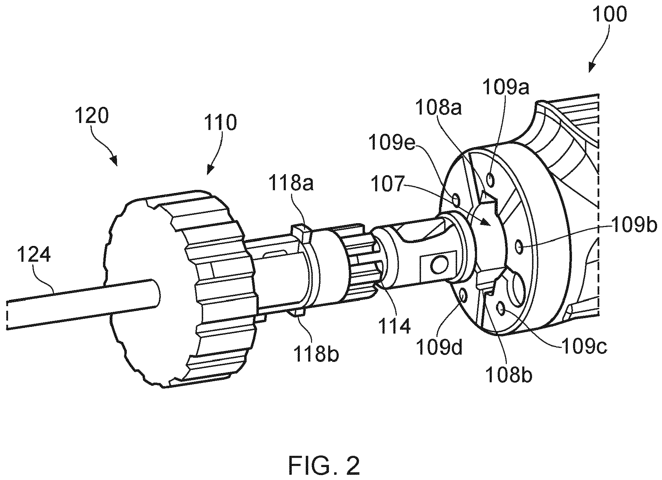

Figure 2 shows an electrosurgical instrument 120 aligned with but not connected to a hand-piece 100. The electrosurgical instrument 120 comprises an elongate shaft 124 having an end effector 15 (see Figure 1) at a distal end and a connection hub 110 at a proximal end. The connection hub 110 includes a connection shaft 114 configured to be received by an axial chamber 107 in the hand-piece 100. The connection hub may comprise one or more locking tabs 118a, 118b corresponding to one or more locking slots 108a, 108b defined in the axial chamber 107, in order to permit insertion of the instrument 120 into the hand-piece 100 in a limited number, preferably one, of orientations about the longitudinal axis of the connection shaft 114.

The hand-piece 100 comprises a plurality of electrical contacts. In the arrangement shown, the hand-piece comprises five electrical contacts 109a, 109b, 109c, 109d, 109e. The electrical contacts 109a-109e are equally spaced from one another and arranged in a circular array around the axial chamber 107. Such electrical contacts 109a-109e are configured to form an electrical connection with corresponding electrical contacts (not shown) on the electrosurgical instrument 120. In the arrangement shown, the instrument's electrical contacts are located on an axial face of the connection hub 110, facing towards the hand-piece's electrical contacts 109a-109e towards the proximal direction of the instrument 120 (i.e. away from the elongate shaft 124).

The surgical device 12 also comprises a sealing member, which will be described in the following. The sealing member is configured to electrically isolate non-corresponding electrical contacts from one another. In other words, when the instrument is connected to the hand-piece, the sealing member permits an electrical contact on the instrument 120 to be electrically connected only to its corresponding electrical contact on the hand-piece 100. The sealing member prevents an electrical connection between separate pairs of electrical contacts. The sealing member may be located between the instrument 120 and the hand-piece 100. The sealing member may be an integral part of the instrument 120. In the arrangement shown in Figure 2, the sealing member may be positioned on an axial face of the instrument 120 between the locking tabs 118a, 118b and the elongate shaft 124.

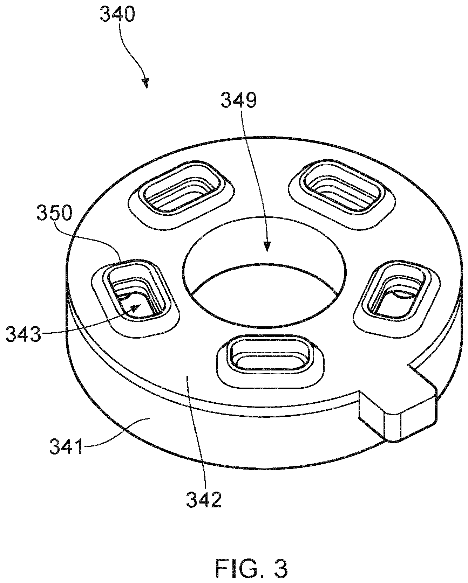

Figure 3 shows an example of a sealing member 340. The sealing member 340 comprises a body portion 341. The body portion 341 is substantially cylindrical and may comprise a central hole 349 configured to receive at least a portion of the connection shaft 114 of the surgical instrument 120. The sealing member 340 may further comprise an interface portion 342, which may be provided as an axial face of the cylindrical body portion 341.

The sealing member 340 further comprises a plurality of apertures 343, each surrounded by a sealing element 350. The apertures 343 may be provided as holes through the thickness of the interface portion 342. The sealing element 350 comprises an elastomeric material such as rubber. The sealing element 350 is substantially rectangular with rounded corners.

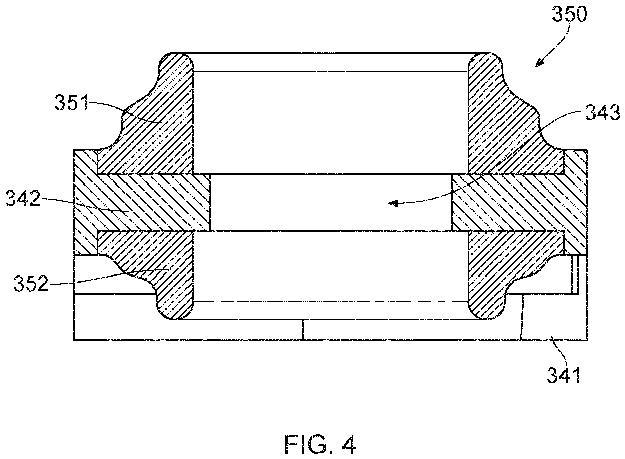

Figure 4 shows a cross-section through a sealing element 350. The sealing element 350 comprises a first portion 351 protruding from a first side of the interface portion 342 and a second portion 352 protruding from a second opposite side of the interface portion 342. When the surgical instrument 120 is connected to the hand-piece 100, the first portion 351 may abut the hand-piece 100 and the second portion 352 may abut the surgical instrument 120. The apertures 343 provide a space in the sealing member 340 through which corresponding electrical contacts on the instrument 120 and the hand-piece 100 may be connected.

The first and second portions 351, 352 have a constant inner diameter and a tiered exterior such that they are wider nearer to the interface portion 342 and narrower furthest from the interface portion 342. When the surgical instrument 120 is connected to the hand-piece 100, the sealing element 350 is compressed in order to form a seal around a pair of corresponding electrical contacts. In the arrangement shown, the first portion 351 must be compressed between the hand-piece 100 and the interface portion 342 in order to connect the surgical instrument 120 to the hand-piece 100, i.e. to fully insert the connection shaft 114 into the axial chamber 107. Similar compression may be required for the second portion 352 of the sealing element 350 in order to assemble the surgical device 12.

The inventor recognised that the force required to connect the surgical instrument 120 to the hand-piece 100 is undesirably high because it relies on the bulk modulus of the elastomeric material from which the sealing elements 350 are formed. In other words, substantially all of the elastomeric material of the sealing elements must be compressed in order to bring the surgical instrument 120 and the hand-piece 100 close enough together for connection. Such a reliance on the bulk compression of the elastomeric material increases the force required for connection and compromises the usability of the surgical device 12. A new sealing member which provides a solution to this problem is described in the following.

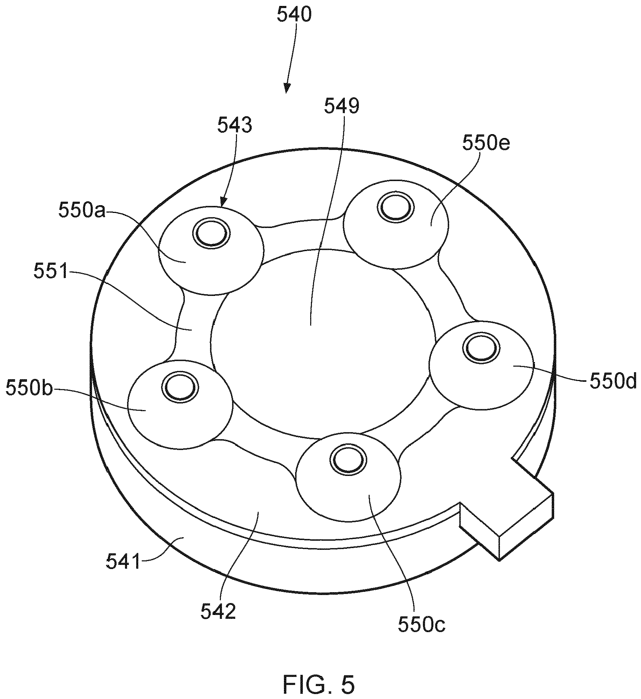

Figure 5 shows an embodiment of a sealing member 540. The sealing member 540 comprises a body portion 541. The body portion 541 is substantially cylindrical and may be formed of an annular wall, which may be an integral part of the surgical instrument 120. The sealing member 540 further comprises an interface portion 542. The interface portion 542 comprises a substantially circular surface arranged perpendicular to an axis of the body portion 541. The sealing member 540 may be an integral part of the surgical instrument 120. The interface portion 542 may form an axial face of the connection hub 110, facing in a direction opposite to that in which the elongate shaft 124 is provided (see Figure 2). The interface portion 542 may comprise a central portion 549 which may receive or be fixed to the connection shaft 114 of the surgical instrument 120.

The sealing member 540 further comprises a plurality of apertures 543. The apertures 543 may be provided as holes through the thickness of the interface portion 542. At least one aperture 543 is surrounded by a sealing element 550. In the arrangement shown, the sealing member 540 comprises five sealing elements 550a, 550b, 550c, 550d, 550e, each one corresponding to one of the pairs of electrical contacts from the surgical instrument 120 and the hand-piece 100. In the arrangement shown, the sealing elements 550a-550e are substantially circular in the plane of the interface portion 542, but it will be understood that other shapes may be adopted. The sealing elements 550a-550e comprise an elastomeric material such as rubber. The sealing elements 550a-550e may consist of an elastomeric material such as rubber.

The sealing elements 550a-550e are arranged in a circular array around a centre of the interface portion 542. The sealing elements 550a-550e may be arranged so as to lie on the vertices of a regular pentagon. The sealing elements 550a-550e may each surround an electrical contact (not shown) of the surgical instrument 120. The sealing elements 550a-550e are configured to be aligned with the electrical contacts 109a-109e (see Figure 2) of the hand-piece 100 when the surgical instrument 120 is connected to the hand-piece 100. Specifically, when the connection shaft 114 is received by the axial chamber 107 and the locking tabs 118a, 118b are aligned with the respective locking slots 108a, 108b, a first sealing element 550a may be aligned with a first electrical contact 109a, a second sealing element 550b may be aligned with a second electrical contact 109b, and so on for each pair of a sealing element with its corresponding electrical contact.

The sealing member 540 may comprise bridge portions configured to connect neighbouring sealing elements 550a-550e in the circular array. For example, the bridge portion 551 in Figure 5 forms a bridge between a first sealing element 550a and a second sealing element 550b adjacent thereto. The bridge portions may comprise an elastomeric material which may be the same material as that from which the sealing elements 550 are formed. In this way, the sealing elements 550a-550e and the bridge portions 551 may be formed from a single moulded component for insertion into the sealing member 540.

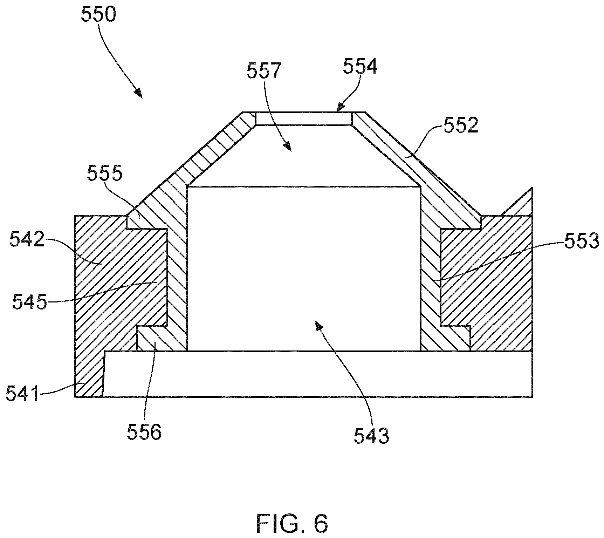

Figure 6 shows a cross-section through a sealing element 550. The sealing element 550 comprises a deflectable portion 552. The deflectable portion 552 protrudes from the surface of the interface portion 542. The deflectable portion 552 comprises an opening 554 at an end furthest from the interface portion 542, i.e. the upper end as shown in Figure 6. In the example in which the sealing element 550 is circular, the inner diameter of the deflectable portion 552 gradually decreases from the interface portion 542 towards the opening 554. Otherwise stated, the deflectable portion 552 is narrower further from the interface portion 542. Similarly, the outer diameter of the deflectable portion tapers uniformly towards the opening 554, i.e. towards the upper end as shown in Figure 6.

This arrangement defines a cavity 557 in the deflectable portion 552. The cavity 557 may have a substantially trapezoidal cross-section. The cavity 557 may be a tapered cavity. The thickness of the wall defining the deflectable portion 552 may be constant over the majority of its depth. The wall thickness may be uniform along at least 50%, preferably at least 70% of the depth of the deflectable portion 552. In the arrangement shown the wall thickness of the deflectable portion 552 is constant along approximately 70% of the depth of the sealing element 550 from the opening 554 to the interface portion 542. The deflectable portion 552 may be substantially frustoconical, that is, the deflectable portion 552 may resemble a cone with a truncated portion. The base of the cone is received by the interface portion 542 and the opening 554 may be located at the truncated portion.

The sealing element further comprises a fixed portion 553 configured to be retained within the sealing member 540. The fixed portion 553 is hollow and sits within the aperture 543. In the arrangement shown, the fixed portion 553 is substantially cylindrical but it will be understood that it may take other shapes corresponding to the shape of the deflectable portion 552. The fixed portion 553 may be formed from the same material as the deflectable portion 552. The fixed portion 553 may be joined to an annular base of the deflectable portion 552 in the region of the interface portion 542. The flexible portion 552 and the deflectable portion 553 may be formed of a single moulded component.

The fixed portion 553 comprises a first flange 555 around an upper end thereof and a second flange 556 around a lower end thereof. The first and second flanges 555, 556 are configured to engage with a collar 545 of the interface portion 542 to secure the sealing element 550 thereto. Although the elastomeric material of the fixed portion 553 is deformable, it is arranged in the sealing member 540 such that in use (i.e. when the surgical instrument 120 is connected, used and then disconnected from the hand-piece 100) the fixed portion 553 is securely retained around the collar 545 of the interface portion 542. In contrast, the shape of the deflectable portion 552 facilitates its deflection when a force is exerted on the upper end thereof, i.e. where the opening 554 is defined. This permits the deflectable portion 552 to deflect in response to a force exerted thereon by the hand-piece 100.

Compared to the sealing element 350 shown in Figure 4, the sealing element 550 in Figure 6 facilitates connection of the surgical instrument 120 to the hand-piece 100 with a lower connection force. Due to the substantially hollow form of the deflectable portion 552 (Figure 6) compared to the first portion 351 (Figure 4) and the uniform thickness of the wall defining the cavity 557, the sealing element 550 can be deformed without requiring any significant compression of the elastomeric material. Instead, the sealing element 550 deforms by way of the deflectable portion 552 deflecting into the cavity 557. Therefore, the cavity 557 facilitates deflection of the sealing element 550 when the surgical instrument 120 is connected to the hand-piece 100. It will be understood that the forces required to facilitate such deflection rely on bending the wall of the deflectable portion 552 instead of a bulk compression of the elastomeric material.

In order to adopt the advantageous sealing member 540 in a surgical device 12, the sealing member 540 may form part of the connection hub 110 of the surgical instrument 120. Each electrical contact of the surgical instrument may be surrounded by a sealing element of the sealing member. An electrical contact may, for example, extend from the surgical instrument 120 (lower end of Figure 6) up through the aperture 543 to a position close to or within the cavity 557. When the surgical instrument is connected with the hand-piece 100, the deflectable portion 552 will deform as the two components are brought together, thereby facilitating an electrical connection between electrical contacts on the instrument 120 and the hand-piece 100.

Various modifications, whether by way of addition, deletion and/or substitution, may be made to all of the above described embodiments to provide further embodiments, any and/or all of which are intended to be encompassed by the appended claims.

## Claims

**1.** A surgical instrument configured to be releasably connectable to a hand-piece, the surgical instrument comprising:
a plurality of electrical contacts configured to be electrically connected to the hand-piece; and
a sealing member comprising a plurality of sealing elements, wherein at least one sealing element of the plurality of sealing elements comprises an elastomeric material and is configured to form a seal around one of the plurality of electrical contacts when the surgical instrument is connected to the hand-piece, such that the electrical contact is electrically isolated from other electrical contacts on the surgical instrument,
wherein the at least one sealing element is configured such that the elastomeric material deflects into a position to form the seal rather than undertaking a bulk compression of the elastomeric material to form the seal.

**2.** The surgical instrument according to claim 1, wherein the sealing element comprises a tapered cavity configured to facilitate deflection of the sealing element when the surgical instrument is connected to the hand-piece.

**3.** The surgical instrument according to claim 1 or claim 2, wherein the sealing element comprises a deflectable portion, configured to deflect when compressed between the surgical instrument and the hand-piece.

**4.** The surgical instrument according to claim 3, wherein the deflectable portion is configured to bend in response to a compression force exerted on the sealing member when the surgical instrument is connected to the hand-piece.

**5.** The surgical instrument according to claim 3 or claim 4, wherein the sealing element comprises an opening through which one of the plurality of electrical contacts is configured to electrically connect to the hand-piece.

**6.** The surgical instrument according to claim 5, wherein the opening is comprised in the narrowest end of the deflectable portion.

**7.** The surgical instrument according to claim 5 or claim 6, wherein the deflectable portion comprises a sloped wall having a thickness, wherein the thickness of the wall is substantially uniform along at least 50%, preferably at least 70%, of the height of the deflectable portion from the opening.

**8.** The surgical instrument according to any of claims 5 to 7, wherein an internal diameter of the deflectable portion decreases gradually towards the opening.

**9.** The surgical instrument according to any of claims 3 to 8, wherein the deflectable portion comprises a frustoconical portion.

**10.** The surgical instrument according to claim 9, wherein the sealing member comprises a substantially cylindrical body portion and the frustoconical portion at least partially protrudes from a flat end face of the cylindrical body portion.

**10.** The surgical instrument according to any preceding claim, wherein the sealing element comprises a fixed portion configured to be mounted within the sealing member, wherein the fixed portion is preferably substantially cylindrical.

**11.** The surgical instrument according to any preceding claim, wherein the surgical device comprises at least five pairs of corresponding electrical contacts, and the sealing member comprises five sealing elements configured to isolate the pairs of corresponding electrical contacts from one another when the surgical instrument is connected to the hand-piece.

**12.** The surgical instrument according to any preceding claim, wherein the sealing member comprising a circular array of sealing elements and at least one bridge portion linking neighbouring sealing elements in the array.

**13.** The surgical instrument according to claim 12, wherein a single elastomeric moulding comprises the sealing elements and the at least one bridge portion.

**14.** The surgical instrument according to any preceding claim, further comprising an end effector for performing surgical operations.

**15.** The surgical instrument according to claim 14, wherein the end effector is disposed at a distal region of the surgical instrument and the sealing member is disposed at a proximal region of the surgical instrument.
